# EUROPEAN PATENT APPLICATION

(11) **EP 1 916 299 A1**
(43) Date of publication of application: **30.04.2008**
(21) Application number: 06745401.7
(22) Date of filing: 12.04.2006
(51) Int. Cl.: C12N 15/00, C12N 5/10, C12N 15/09, C12P 21/02

(54) **DNA, VECTOR, TRANSFORMANT AND METHOD FOR PRODUCING APA PROTEIN**

(30) Priority: 20.06.2005 JP 2005179766
(71) Applicant: P-Lap Corporation, Nagoya-shi, Aichi 465-0032 (JP)
(72) Inventor: MIZUTANI, Shigehiko, c/o P-LAP Corporation, Nagoya-shi, Aichi 465-0032 (JP)
(74) Representative: Oser, Andreas
(86) International application number: PCT/JP2006/307748
(87) International publication number: WO 2006/137209

(57) **Abstract**

It is intended to provide a production method by which human APA protein can be recovered more efficiently and in a larger quantity compared to a conventional method; DNA encoding a recombinant human APA protein which is necessary when the human APA protein is produced; a vector in which the DNA is integrated; and a transformant which retains the vector.

In order to recover an active site of the human APA protein (a site in the human APA protein effective in therapy or the like of preeclampsia) in a large quantity and easily, the recombinant human APA protein (hereinafter referred to as a secretory APA) in which the amino acid sequence from an N terminal to a 71st residue has been replaced with human trypsin II represented by SEQ ID No: 3 or the signal peptide of human A-LAP represented by SEQ ID NO: 4 is cultured.

## Description

### BACKGROUND OF THE INVENTION

The present invention relates to a method for producing a human APA protein efficiently, and a DNA encoding a recombinant human APA protein which is necessary when the human APA protein is produced, a vector in which the DNA is integrated, and a transformant which retains the vector.

### Description of the Background Art

A human APA protein has been known as an active ingredient which is predicted to be effective for treatment of preeclampsia. The human APA protein can be recovered from human placenta. However, the operations to recover the human APA protein from human placenta are difficult in themselves. In addition, a fraction of a human APA protein is contained in human placenta. Therefore, traces of a human APA protein can be recovered from human placenta. In other words, it can be said that a human APA protein is a protein which is quite difficult to be recovered in nature. With this reason, the study in which a recombinant human APA protein is recovered by culture has been conducted. As one example thereof, the method described in Non-patent Reference 1, in which a human APA protein is cultured and recovered using a hamster cell, has been known.

In a case where a human APA protein is cultured using such a hamster cell, 0.06 mg of human APA proteins with respect to 300 mL of cultures can be recovered.

Non-patent Reference 1: Gilles VAZEUX, Sherwin WILK, Elizabeth WILK, Pierre CORVOL and Catherine LLORENS-CORTES, "Production and properties of a recombinant soluble form of aminopeptidase A", Eur. J. Biochem., 1998, 254, p.671-678

### SUMMARY OF THE INVENTION

However, a recovery rate of human APA proteins is still low and the current situation is that even the amount needed for various experiments is hardly obtainable. Therefore, a method for recovering a human APA protein which can be recovered more efficiently and in a large amount is desired.

The object of the present invention is to provide a method for producing a human APA protein which can be recovered more efficiently and in a larger amount as compared to the conventional method, and a DNA encoding a recombinant human APA protein which is necessary when the human APA protein is produced, a vector in which the DNA is integrated, and a transformant which retains the vector.

A first aspect of the present invention relates to a DNA encoding a recombinant human APA protein shown in SEQ ID NOS: 1 and 2.

A second aspect of the present invention relates to a DNA including a DNA encoding a recombinant human APA protein shown in SEQ ID NOS: 1 and 2.

A third aspect of the present invention relates to a DNA, wherein a part of the DNA which encodes a recombinant human APA protein shown in SEQ ID NOS: 1 and 2 is replaced, deleted, or added.

A fourth aspect of the present invention relates to a vector in which the DNA according to any one of the first, second or third aspect of the present invention is integrated.

A fifth aspect of the present invention relates to a transformant which retains the vector according to the fourth aspect of the present invention.

A sixth aspect of the present invention relates to the transformant according to the fifth aspect of the invention, wherein the transformant is an insect cell.

A seventh aspect of the present invention relates to a method for producing a human APA protein, the method including culturing the transformant according to the fifth and sixth aspects of present the invention, and recovering a resultant expression product thereof.

According to the present invention, 71 amino acids from an N-terminal are replaced by a signal peptide represented by SEQ ID NO: 3 (which corresponds to SEQ ID NO: 1) and SEQ ID NO: 4 (which corresponds to SEQ ID NO: 2), which are transcribed and translated from DNAs shown in SEQ ID NOS: 1 and 2, thereby the human APA protein is changed from an original cell membrane-bound form into an extracellular secretion form. As a result, an expression rate of a recombinant human APA protein extremely increases, compared to a conventional method, and a human APA protein (an active site) can be recovered very efficiently and in a large amount.

Furthermore, by culturing the above-described human APA protein with a baculovirus expression system using an insect cell, 10mg to 30mg of human APA protein active sites (which is 10 times or more than a conventional recovery rate) with respect to 3L of cultures can be recovered.

As one embodiment of the present invention, a description will be made on a method for producing a human APA protein, and DNA encoding a recombinant human APA protein which is necessary when the human APA protein is produced, a vector in which the DNA is integrated, and a transformant which retains the vector.

The present inventor has found that, in order to recover a human APA protein active site (a site of a human APA protein, which is effective for treatment of preeclampsia and the like) in a large amount and without difficulty, a recombinant human APA protein where the amino acid sequence from an N terminal to a 71st residue thereof has been replaced with human trypsin II represented by SEQ ID NO: 3 or a signal peptide of human A-LAP represented by SEQ ID NO: 4 (hereinafter, referred to as a secretory APA) should have been cultured.

Moreover, the present inventor has also found that, a secretory APA can be expressed very easily and in a large amount using an insect cell such as a cell of silkworm when the secretory APA is cultured. Since the signal peptide described above is quickly cleaved after synthesis of protein and moving to the secretion pathway in the cell, only an active site can easily be recovered.

Now, a method for producing a human APA protein (an active site) will be described below in detail. The method uses an insect cell to culture using such a secretory APA. Out of the above secretory DNAs, the method of a case where the sequence shown by SEQ ID NO: 1 is replaced to the trypsin II signal peptide will be described below in detail. However, substantially the same applies to a case where the sequence shown by SEQ ID NO: 2 is replace to the human A-LAP signal peptide.

### (a) Amplification of cDNA which encodes a secretory APA and construction of transfer vector

A construction of cDNA which encodes a secretory APA is carried out by the PCR method in which cDNA encoding a naturally occurring human APA protein is used as a template. PCR (Polymerase Chain Reaction) is a reaction to specifically amplify a DNA fragment sandwiched between a set of primers by performing continuous and concatenate DNA Polymerase reaction.

The sequence here used is as follows: the primer sequence of tropomyosin of a lobster (AACTCCTAAAAAACCGCCACC), the human trypsin II signal sequence (MNLLLILTFVAAAVAA), and sense strand primer designed to encode Ala⁷²-Asp⁷⁶ of a human APA; and anti-sense strand primer designed to encode Leu⁹⁵³-Gly⁹⁵⁷ of a human APA, six histidine residues, and termination codon. For the anti-sense strand primer, 30 cycles consisting of 30 sec at 94 °C, 30 sec at 52 °C, and 3 min at 72 °C as one cycle are repeatedly performed and the target DNA fragments. The amplified DNA fragments are separated by using agarose gel electrophoresis and target DNA fragments are recovered and purified from gel by a conventional method.

Then, the purified DNA fragments are integrated into pENTR plasmid using pENTR-/D-TOPO cloning kit from Invitrogen Corporation. Furthermore, cDNAs of the secretory APA are integrated into pDEST8 (Invitrogen Co.) which is the transfer vector for baculovirus by Geteway LP Clonase from Invitrogen Corporation.

As described above, the amplification of cDNA encoding secretory APA, and the construction of transfer vector are carried out.

### (b) Production of secretory APA expressing baculovirus

Since E. coli is a prokaryote, the situation that the proper folding cannot be obtained when a protein is expressed using an expression system derived from an eucaryote, such as animals, often occurs. With this reason, the present inventor has paid attention to baculovirus-insect cell system as a large-scale expression system for eukaryotic cells. The baculovirus expression system is much inexpensive than a mammalian cell system. The operations or the like relating to culture are not burdensome and usability thereof is good. The characteristics of the baculovirus expression system enable to express a target protein in a large amount while keeping primary biological characterization.

In the baculovirus expression system, for example, in the field of recombinant protein expression, baculovirus refers to double-stranded DNA virus of insects called Nuclear Polyhedorosis Virus (NPV). The virus belonging to this group has characteristics of producing a large amount of inclusion bodies called polyhedra which are consisted of proteins called polyhedrin in the nuclei of infected cells. The baculovirus expression system is a system to synthesize a recombinant protein using a very strong promoter in the polyhedrin gene within insect cells.

As the baculovirus expression system described above, now commonly used systems are systems using Autographa californica NPV (AcNPV) which belongs to Noctuidae as a vector and Sf9 derived from embryo of Spodoptera Frugiperda which also belongs to Noctuidae as an infected cell. The system using BmNPV Nuclear Polyhedorosis Virus BmNPV derived from Bombyx mori is developed in Japan. The latter system using Bombyx mori has an advantage in that a large number of products can be obtained by directly inoculating recombinant virus into embryo of Bombyx mori, without need of performing culture in large volume using culture medium containing expensive serum. Either system is applicable for the present invention without limitation.

Furthermore, the production of recombinant virus will be explained. As a baculovirus gene is as large as 30 kb, it is impossible for common in vitro DNA manipulation to directly insert the gene to be expressed downstream of a polyhedrin promoter. However, Bac-to-Bac system of Invitrogen Co. permits simple and quick production of recombinant virus. That is, as described above, baculovirus DNA containing cDNA of secretory APA was produced by the following procedures. The target DNA fragment is integrated in a plasmid (transfer vector). Then, target DNA fragment is inserted into the E. coli DH10Bac (Invitrogen Co.).

In the Bac-to-Bac system, a gene to be expressed is firstly integrated into a donor plasmid having the sequences of both end regions of the E. coli transposon Tn7. Then, the integrated gene is inserted into E. coli DH10Bac containing baculovirus DNA (baculovirus shuttle vector = bacmid) in which Tn7 target site and replication origin region of E. coli are integrated. A helper plasmid expressing transposase is also integrated in the E. coli DH10Bac. The target gene-incorporated region of the donor plasmid in E. coli is transferred to bacmid by an action of transposase, thereby a recombinant bacmid can be obtained. The bacmid is purified from E. coli and transfected into an insect cell, whereby virus being produced in the insect cell. The method has an advantage in that the insertion of an exogenous gene is performed within E. coli, the reduction of time can be achieved. Another advantage is that, since the virus is cloned at this stage, purification of the target virus, with the exception of non-recombinant virus, do not needed.

A virus is produced by the following procedure. Bacmid DNA of a human secretory APA thus obtained is transfected into Sf9 cell using Cellfection reagent (Invitrogen Co.), and then the cell was cultured at 27 °C for three days. Then, the viruses secreted in culture supernatant are recovered and the high titer virus liquid was produced after two times of amplification.

As described above, the production of baculovirus expressing secretory APA is performed.

### (c) Expression of human APA protein

The recombinant virus obtained by the operation described above is scaled up to increase, and the high titer virus stock is obtained. The passage number at the time of scaling up should be at most six, because, if the number of passage is largely increased, mutants are expressed and the expression efficiency decreases. The virus stock obtained can be semipermanently stored at -80 °C, however, freezing and thawing lead to decrease of titer. Therefore, since the virus stock can be stored at 4 °C for around 2 years, it is better not to freeze the portion to be used.

In order to express proteins efficiently, the infection of a cell with as many viruses as possible is necessary. For this reason, multiphcity of infection (MOI) is around 5 to 10. The cell is infected during the log growth phase, rather than infected after the density of the cells increases too much. Although the expression of secretory APA generally reaches the peak 48 to 72 hours after infection, time-course observation is carried out.

In the present embodiment, Sf9 cells are cultured at 27 °C in the serum-free culture medium SFM900II (Invitrogen Co.) using a 3L spinner flask which can control the enzyme concentration to be 8 ppm. After the cell concentration has reached 1.5 × 10⁶ cells/mL, the high titer virus liquid produced as described above is added in the flask so that the ratio of viruses to cells becomes substantially 10 to 1 (MOI=10). Furthermore, culture is continued for three days after virus infection, and the expression of proteins is performed.

As described above, the expression of the human APA protein is carried out.

### (d) Purification of human APA protein

3L of above culture solution is recovered and cells and the culture supernatant are separated by a centrifugal separator. The cell culture supernatant containing the separated human APA protein is dialyzed with Tris-HCL solution (pH 8.0) with 50mM at 4 °C for 36 hours. Affinity chromatography is used after artificial dialysis treatment.

After purification is performed by the anion-exchange column chromatography (DEAE-Toyoperl: Tosoh Corp.), purification by metal-chelate column chromatography using cobalt ion (Chelating Sepharose: Amasham Biosciences) is performed, then human APA proteins are harvested. If further necessary, the purification can be carried out by common chromatography such as gel filtration chromatography and hydrophobic chromatography.

As described above, the purification of human APA proteins is performed.

According to the method for producing human APA protein described above, since baculovirus expression system uses an insect cell which is an eucaryote, even in the case of proteins derive from an eucaryote, the resultant products, which is thought to have proper folding, can be obtained in many cases, and disulfide binding is made similar to the native form. Modification after labored translation can be expected in E. coli, and phosphorylation of expression product and addition of aliphatic acid occur. A signal peptide is correctly recognized and cut, and secretion occurs. Concerning glycosylation, Asn-linked oligosaccharide is not processed to combined oligosaccharide and turns into high-mannose oligosaccharide. Tkm/Ser-linked oligosaccharide is said not to be added. Therefore, molecular weight of the protein (the oligosaccharide site) expressed in the baculovirus system tends to be slightly smaller than that of naturally occurring type, and the protein expressed in the baculovirus system is more suitable for crystallization than the naturally occurring type protein.

In addition, 10mg to 30mg of human APA protein active sites (which is 10 times or more than a conventional recovery rate) with respect to 3L of cultures can be recovered.

## Claims

1. A DNA which encodes a recombinant human APA protein shown in SEQ ID NOS: 1 and 2.

2. A DNA comprising a DNA which encodes a recombinant human APA protein shown in SEQ ID NOS: 1 and 2.

3. A DNA, wherein a part of the DNA which encodes a recombinant human APA protein shown in SEQ ID NOS: 1 and 2 is replaced, deleted, or added.

4. A vector in which the DNA according to any one of claims 1 to 3 is integrated.

5. A transformant which retains the vector according to claim 4.

6. The transformant according to claim 5, wherein the transformant is an insect cell.

7. A method for producing a human APA protein, the method comprising culturing the transformant according to any one of claims 5 to 6, and recovering a resultant expression product.
